(19) Europäisches Patentamt
European Patent Office
Office européen des brevets

(11) **EP 3 928 690 B1**

(12) **EUROPEAN PATENT SPECIFICATION**

(45) Date of publication and mention
of the grant of the patent:
**11.09.2024 Bulletin 2024/37**

(21) Application number: **20182512.2**

(22) Date of filing: **26.06.2020**

(51) International Patent Classification (IPC):
**A61B 5/02** (2006.01)  **A61B 5/026** (2006.01)
**A61B 5/0295** (2006.01)  **A61B 5/00** (2006.01)

(52) Cooperative Patent Classification (CPC):
**A61B 5/02007; A61B 5/0261; A61B 5/0295;**
**A61B 5/4035; A61B 5/4815; A61B 5/4818;**
A61B 5/6826; A61B 2505/07

(54) **METHOD AND APPARATUS FOR ASSESSING PERIPHERAL ARTERIAL TONE**

VERFAHREN UND VORRICHTUNG ZUR BEURTEILUNG DES PERIPHEREN ARTERIELLEN TONUS

PROCÉDÉ ET APPAREIL D'ÉVALUATION DU TONUS ARTÉRIEL PÉRIPHÉRIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**29.12.2021 Bulletin 2021/52**

(73) Proprietor: **Ectosense NV**
**3110 Rotselaar (BE)**

(72) Inventors:
• **GEEURICKX, Wout**
**3010 Kessel-Lo (BE)**
• **MASSIE, Frederik**
**3000 Leuven (BE)**
• **VITS, Steven**
**1060 Sint-Gillis (BE)**
• **VAN PEE, Bart**
**3000 Leuven (BE)**

(74) Representative: **IP HILLS NV**
**Bellevue 5/501**
**9050 Gent-Ledeberg (BE)**

(56) References cited:
**US-A1- 2006 009 700**

• **MEIR NITZAN ET AL: "The variability of the photoplethysmographic signal - a potential method for the evaluation of the autonomic nervous system", PHYSIOLOGICAL MEASUREMENT, INSTITUTE OF PHYSICS PUBLISHING, BRISTOL, GB, vol. 19, no. 1, 1 February 1998 (1998-02-01), pages 93 - 102, XP020073825, ISSN: 0967-3334, DOI: 10.1088/0967-3334/19/1/008**
• **THOMAS PENZEL ET AL: "Peripheral arterial tonometry, oximetry and actigraphy for ambulatory recording of sleep apnea; Watch-PAT for sleep apnea recording", PHYSIOLOGICAL MEASUREMENT, INSTITUTE OF PHYSICS PUBLISHING, BRISTOL, GB, vol. 25, no. 4, 1 August 2004 (2004-08-01), pages 1025 - 1036, XP020074176, ISSN: 0967-3334, DOI: 10.1088/0967-3334/25/4/019**
• **SUZUKI T ET AL: "Development of a sleep apnea event detection method using photoplethysmography", 2010 ANNUAL INTERNATIONAL CONFERENCE OF THE IEEE ENGINEERING IN MEDICINE AND BIOLOGY SOCIETY : (EMBC 2010) ; BUENOS AIRES, ARGENTINA, 31 AUGUST - 4 SEPTEMBER 2010, IEEE, PISCATAWAY, NJ, USA, 31 August 2010 (2010-08-31), pages 5258 - 5261, XP032108321, ISBN: 978-1-4244-4123-5, DOI: 10.1109/IEMBS.2010.5626291**

- **PENZEL THOMAS ET AL: "New Methods for the Non-Invasive Assessment of Sympathetic Activity During Sleep. Neue Methoden zur nicht-invasiven Erfassung des Sympathikotonus im Schlaf", SOMNOLOGIE, vol. 6, no. 2, 1 May 2002 (2002-05-01), DE, pages 69 - 73, XP093122828, ISSN: 1432-9123, DOI: 10.1046/j.1439-054X.2002.02178.x**

## Description

### Technical Field

The present invention generally relates, amongst others, to a method and an apparatus for assessing Peripheral Arterial Tone or PAT. More particularly, it relates to robustly monitoring peripheral arterial tone for detecting for example sleep-related events.

### Background

During a cardiac cycle of an individual, i.e. the period between two heart beats, blood is pumped through the vascular system of the individual in a pulsating fashion. In other words, during a cardiac cycle, the volume of blood in for example a finger, a nostril, an ear, a forehead, the inside of a mouth, a toe, a wrist, an ankle, etc., increases and decreases cyclically. The blood comprised in arteries is called arterial blood. The blood comprised in veins is called venous blood.

A common method for measuring this fluctuation in blood volume is optical plethysmography, or photoplethysmography, during which an optical plethysmogram, or PhotoPlethysmoGram, or PPG, is used to detect changes in blood volume in the microvascular bed of the tissue. A PPG is often obtained by shining light from one or more light sources, such as for example LEDs, onto an investigated volume, and detecting collected light corresponding to the light being reflected or transmitted in the investigated volume on a sensor, wherein the sensor may for example comprise or correspond to a photodetector, such as for example a photodiode. The light sources and the sensor can for example be arranged on opposite sides of a finger of an individual, hereby allowing the measurement of a transmission mode PPG, or at the same side of a finger of the individual, hereby allowing the measurement of a reflectance mode PPG. With each cardiac cycle the heart pumps arterial blood to the investigated volume. The physical events corresponding to a change in arterial blood volume in the investigated volume for example in a cardiac cycle can be captured by optical plethysmography. For example, US20060009700A1 describes an apparatus and method for the non-invasive evaluation, detection, and monitoring of a physiological state or medical condition by assessing peripheral circulation. For example, the scientific publication by Nitzan et al. published in the Physiol. Meas. 19 (1998) 93-102 and entitled "The variability of the photoplethysmographic signal - a potential method for the evaluation of the autonomic nervous system" describes that the variability of the PPG parameters shows promise for the assessment of the function of the autonomic nervous system.

In addition to cyclic fluctuations in arterial blood volume, the arterial blood volume in the tissue is also influenced by the diameters of small arteries, or arterioles, in the investigated volume. These arterioles have muscular walls which can contract to reduce the diameter arterioles. In other words, when these arterioles contract and reduce in diameter, the volume of blood comprised in the corresponding arterioles evidently reduces. Optical plethysmography is a measurement technique which can be used to monitor changes in the volume of blood comprised in arterioles when these arterioles contract in diameter. Monitoring changes in the arterial blood volume with optical plethysmography therefore empirically provides information on relative changes in muscle tension or 'tone' of the smooth muscle tissue of the arterioles, also referred to as Peripheral Arterial Tone or PAT.

Because arterial blood flow to the investigated volume can be modulated by multiple other physiological phenomena, optical plethysmography can also be used to monitor breathing, hypovolemia, other circulatory conditions, and for example can be used for diagnosing sleep disorders. Sleep disorder diagnosis is a medical field wherein a patient's sleep is monitored during a certain time, e.g. one or more nights. Based on the monitoring, different sleep-related events may be identified such as for example apneic events, snoring, or limb movements.

The reuptake of breathing at the end of a sleep apnea usually coincides with an adrenaline release. Adrenaline is released in the bloodstream and binds to adrenergic receptors in the arterioles in the investigated volume, which triggers an increase in muscle tension of the arterioles resulting in a reduction of the arteriole diameter and a reduction of arterial blood volume in the investigated volume. Monitoring Peripheral arterial tone through for example optical plethysmography can therefore provide valuable information on the occurrence of sleep-related events such as for example sleep apnea. Several scientific publications widely report that a signal acquired by optical plethysmography provides information on changes in arterial tone through changes in the amplitude of the pulses in the signal. For example, the scientific publication entitled "Effect of pain on autonomic nervous system indices derived from photoplethysmography in healthy volunteers" by Hamunen et al. published in the British Journal of Anesthesia, Volume 108, Issue 5, P838-844, on May 01, 2012, reports that the Pulse PlethysmoGraphic Amplitude, or PPGA, of the photoplethysmograph is due to pulsatile changes in tissue volume, mainly arterial blood and that during sympathetic activation, or vasoconstriction, PPGA decreases.

The article "Peripheral arterial tonometry, oximetry and actigraphy for ambulatory recording of sleep apnea" by Thomas Penzel et al. published in the Physiol. Meas. 25 (2004) 1025-1036, describes PAT analysis for sleep apnea.

EP 3 928 690 B1

However, monitoring changes in the amplitude of pulses in the signal acquired by optical plethysmography does not accurately reflect changes in arterial tone. In other words, considering the amplitude of pulses in the signal and monitoring changes in this amplitude does not allow to accurately determine changes in sympathetic tone.

**Summary**

It is thus an object of embodiments of the present invention to propose a computer-implemented method and an apparatus which do not show the inherent shortcomings of the prior art. More specifically, it is an object of embodiments of the present invention to propose a method and an apparatus to accurately and robustly determine changes in peripheral arterial tone by optically measuring changes in the arterial blood volume in the investigated volume.

The scope of protection sought for various embodiments of the invention is set out by the independent claims.

The embodiments and features described in this specification that do not fall within the scope of the independent claims, if any, are to be interpreted as examples useful for understanding various embodiments of the invention.

There is a need for accurately and robustly determining changes in the monitored arterial blood volume on the optical measurement of peripheral arterial tone.

This object is achieved, according to a first example aspect of the present disclosure, by a computer-implemented method according to claim 1.

The computer-implemented method according to the present disclosure allows determining peripheral arterial tone in an accurate and robust manner. By determining a logarithm or a function approximation thereof of a function of the light intensities, the determination of changes in arterial blood volume in the investigated volume between two points in time with the computer-implemented method according to the present disclosure is more accurate than by monitoring changes in amplitude of pulses in the optical plethysmography signal. Therefore, with the computer-implemented method according to the present disclosure, it becomes possible to assess PAT of an individual more accurately and more robustly than with existing prior art solutions. In other words, the resulting evaluation of changes in arterial blood volume in the investigated volume through the determination of a logarithm or a function approximation thereof of a function of the light intensities therefore provides a more accurate and robust assessment of peripheral arterial tone of the individual.

In the context of the present disclosure, an investigated volume of an individual is for example a volume defined in an investigated tissue of the individual which is monitored by optical plethysmography and in which light emitted by optical plethysmography propagates and is collected on a sensor for optical plethysmography. In other words, an investigated volume of an individual is for example a volume defined in an investigated tissue of the individual for which an optical plethysmography signal is acquired. For example, the investigated volume is a peripheral tissue volume of the individual. For example, the investigated volume is a volume defined in finger, a tip of a finger, a distal end of a digit of the individual, a nostril, an ear, a forehead, the inside of a mouth, a toe, a tip of a toe, a wrist, an ankle of the individual. In the context of the present disclosure, the investigated volume of an individual comprises the skin of the individual comprised in the investigated volume and further comprises the blood volume present in the investigated volume. In the context of the present disclosure, peripheral arterial tone is understood as arterial tone changes in investigated arterial beds in the investigated volume of an individual. In other words, determining pulsatile volume changes in the vascular beds of the investigated volume of the individual allows determining or assessing information indicative for muscle tension or 'tone' of the smooth muscle tissue of the arterioles in the investigated volume and therefore allows determining or assessing peripheral arterial tone which is modulated by the sympathetic nervous system. Determining peripheral arterial tone is non-invasive and can for example be used to detect heart diseases, erectile dysfunction, sleep apnea, obstructive sleep apnea, cardiovascular conditions, etc..

In the context of the present disclosure, an optical plethysmography signal is a signal measured by optical plethysmography. For example, the optical plethysmography signal is an optical plethysmogram. For example, the optical plethysmography signal is a PPG. The optical plethysmography signal is for example measured at the tip of a finger of the individual by an optical plethysmography setup comprising at least one light source and a sensor. In the context of the present disclosure, a light intensity corresponds to the intensity of light collected on the sensor of the optical plethysmography setup, wherein the light collected on the sensor corresponds to light generated by one or more light sources being transmitted through or reflected in the investigated volume of the individual.

In the context of the present disclosure, the oxygen saturation estimate or $SpO_2$ or hemoglobin composition corresponds to a fraction of oxygenated hemoglobin related to a total amount of hemoglobin in the arterial blood volume in the investigated volume. For example, the oxygen saturation estimate or $SpO_2$ or hemoglobin composition corresponds to a ratio of the concentration of oxygenated hemoglobin on the sum of the concentrations of oxygenated and deoxygenated hemoglobin in the arterial blood volume being monitored in the investigated volume. Alternatively, the oxygen saturation estimate or $SpO_2$ or hemoglobin composition corresponds to a ratio of the volume fraction of oxygenated hemoglobin on the sum of the volume fractions of oxygenated and deoxygenated hemoglobin in the arterial blood volume being monitored in the investigated volume.

In the context of the present disclosure, deoxygenated hemoglobin is defined as the form of hemoglobin without

4

the bound oxygen, and without any other bound molecule such as for example carbon monoxide, carbon dioxide, or iron. In the context of the present disclosure, oxygenated hemoglobin is defined as the form of hemoglobin with the bound oxygen. In the context of the present disclosure, light emitted by the light sources of an optical plethysmography setup comprises photons which reach the sensor through a probabilistic path of one or multiple scattering events. This optical path is not straight and is often assumed to follow a curved spatial probability distribution. The investigated volume along this curved optical path forms the volume which is sampled or investigated by optical plethysmography. The computer-implemented method according to the present disclosure evaluates one or more changes in arterial blood volume in the investigated volume between the two or more points in time and hereby assessing PAT of the individual. In the context of the present disclosure, a change in arterial blood volume in the investigated volume between two points in time corresponds to a relative change between the volume of arterial blood present in the investigated volume at a first point in time and the volume of arterial blood present in the investigated volume at a second point in time different from the first point in time. The blood comprised in arteries is called arterial blood. The blood comprised in veins is called venous blood.

In the context of the present disclosure, a chromophore is a molecular unit which absorbs or scatters light in the investigated volume. For example, in the context of the present disclosure, examples of chromophores are melanin molecules, oxygenated hemoglobin, deoxygenated hemoglobin, etc. In the investigated volume, the decay in light intensity of the incident light emitted by a light source of an optical plethysmography setup follows the Beer-Lambert law which can be formulated as in equation (1):

$$I = I_0 e^{-d \sum_i \varepsilon_i V_i - G(\lambda)} \tag{1}$$

wherein:

- $I_0$ corresponds to the intensity of incident light emitted by a light source of an optical plethysmography setup;
- $\varepsilon_i$ comprises an absorption coefficient and/or a scattering coefficient and/or an extinction coefficient of chromophore $i$;
- $V_i$ corresponds to either a volume fraction or a concentration of chromophore $i$ in the investigated volume;
- $d$ corresponds to the optical path length, wherein the optical path length corresponds to the path length a photon travels along before reaching the sensor of an optical plethysmography setup, wherein the optical path length is a function of, among others, the wavelength of the incident light and of chromophore composition in the investigated volume, and wherein the optical path length is dependent on the distance between the light source emitting the photon and the sensor. From the publication of Chatterjee et al. entitled "Monte Carlo Analysis of Optical Interactions in Reflectance and Transmittance Finger Photoplethysmography", published in Sensors (Basel) 19(4):789 on February 15 2019, doi:10.3390/s19040789, for a distance between the light source emitting the photons and the sensor of a few millimetres, such as for example 3 mm or less than 3mm, it can be approximated that the optical path length can be assumed constant;
- $G$ corresponds to a scattering dependent light intensity loss parameter and which depends on the wavelength $\lambda$. of the incident light emitted by the light source.

Considering the following parameters:

- $V_{i,d}$ corresponding to either a volume fraction or a concentration of chromophore i in the investigated volume at a first point in time along the optical plethysmography signal;
- $V_{i,s}$ corresponding to either a volume fraction or a concentration of chromophore i in the investigated volume at a second point in time along the optical plethysmography signal;
- $I_h$ corresponding to the light intensity measured by the sensor of the optical plethysmography setup at the first point in time;
- $I_l$ corresponding to the light intensity measured by the sensor of the optical plethysmography setup at the second point in time;

the Beer-Lambert law as formulated in equation (1) can be evaluated at the first point in time and at the second point in time. When taking a ratio of both expressions, equation (2) is obtained:

$$\frac{I_l}{I_h} = e^{-d \sum_i \varepsilon_i (V_{i,s} - V_{i,d})} \tag{2}$$

Then, equation (3) is obtained by taking the natural logarithm of both sides of equation (2), as follows:

$$ln\left(\frac{I_l}{I_h}\right) = -d\sum_i \varepsilon_i(V_{i,s} - V_{i,d}) \qquad (3)$$

If a logarithm with another base b than Euler's number e is used, equation (3) then becomes:

$$log_b\left(\frac{I_l}{I_h}\right) = \frac{-d\sum_i \varepsilon_i(V_{i,s} - V_{i,d})}{ln(b)} \qquad (3')$$

It can be seen that the above equation (3') is equal to equation (3) up to a constant $\frac{1}{ln(b)}$ .

Equation (3) can be simplified to equation (4) when when the difference $V_{i,s}$ - $V_{i,d}$ is being renamed $\Delta V_i$, thereby obtaining:

$$ln\left(\frac{I_l}{I_h}\right) = -d\sum \varepsilon_i \Delta V_i \qquad (4)$$

From equation (4), it can be seen that the logarithm of the fraction of light intensities at the first point in time and at the second point in time is linearly related to the difference in either volume fraction or concentration of chromophore i between the first point in time and the second point in time.

Some chromophores remain attached to the epidermis of the individual between the two points in time along the optical plethysmography signal. For example, melanin molecules remain fixed to the investigated volume between the two points in time along the optical plethysmography signal. The difference in either volume fraction or concentration of such chromophores, such as for example melanin molecules, between the two points in time is therefore null. The contribution to the right-hand side of equation (4) of such chromophores is therefore null.

The main chromophores of which either the volume fraction or the concentration fluctuates between the two points in time along the optical plethysmography signal are the oxygenated and deoxygenated hemoglobin in the arterial blood volume. The two predominant forms of hemoglobin in the context of the present disclosure, i.e. oxygenated hemoglobin and deoxygenated hemoglobin, demonstrate absorption and scattering coefficients which are significantly different for most wavelengths of light.

The effect of all other chromophores, wherein all other chromophores are not oxygenated hemoglobin nor deoxygenated hemoglobin, of which either the volume fraction or the concentration fluctuates between the two points in time along the optical plethysmography signal can be written as the product of their combined extinction coefficient, $\varepsilon_{other}$, and one minus the sum of either the volume fractions or the concentrations of oxygenated hemoglobin and deoxygenated hemoglobin, wherein the sum of either all volume fractions or all concentrations equals 1.

Taking the above considerations into account, equation (4) can then be rewritten as follows into equation (5):

$$ln\left(\frac{I_l}{I_h}\right) = -d\left(\varepsilon_{HbO_2}\Delta V_{HbO_2} + \varepsilon_{Hb}\Delta V_{Hb}\right.$$

$$\left. + \varepsilon_{other}\left(1 - \left(V_{Hb,s} + V_{HbO_2,s}\right) - \left(1 - \left(V_{Hb,d} + V_{HbO_2,d}\right)\right)\right)\right)$$

$$ln\left(\frac{I_l}{I_h}\right) = -d\left(\varepsilon_{HbO_2}\Delta V_{HbO_2} + \varepsilon_{Hb}\Delta V_{Hb} + \varepsilon_{other}\left(-(\Delta V_{Hb} + \Delta V_{HbO_2})\right)\right)$$

$$ln\left(\frac{I_l}{I_h}\right) = -d\left(\varepsilon_{HbO_2}\Delta V_{HbO_2} + \varepsilon_{Hb}\Delta V_{Hb} - \varepsilon_{other}\left(\Delta V_{Hb} + \Delta V_{HbO_2}\right)\right)$$

$$ln\left(\frac{I_l}{I_h}\right) = -d\left(\left(\varepsilon_{HbO_2} - \varepsilon_{other}\right)\Delta V_{HbO_2} + (\varepsilon_{Hb} - \varepsilon_{other})\Delta V_{Hb}\right) \qquad (5)$$

It is known that the oxygen saturation estimate can be defined according to equation (6):

$$\frac{V_{HbO_2}}{V_{HbO_2} + V_{Hb}} = SpO_2 \tag{6}$$

wherein:

- $V_{HbO_2}$ corresponds to either the volume fraction or the concentration of oxygenated hemoglobin comprised within the arterial blood in the investigated volume;
- $V_{Hb}$ corresponds to either the volume fraction or the concentration of deoxygenated hemoglobin comprised within the arterial blood in the investigated volume.

Additionally, $V_{blood}$ is defined as the total volume fraction or the total concentration of oxygenated and deoxygenated hemoglobin comprised within the arterial blood in the investigated volume and is defined as follows in equation (7):

$$V_{HbO_2} + V_{Hb} = V_{blood} \tag{7}$$

It is assumed that under normal circumstances, the sum of the volume fractions or of the concentrations of oxygenated and deoxygenated hemoglobin within the arterial blood volume or the sum of the concentrations of oxygenated and deoxygenated hemoglobin within the arterial blood volume remains approximately constant throughout the measurement of the optical plethysmography signal. Indeed, only the ratio of oxygenated hemoglobin and deoxygenated hemoglobin, i.e. only the oxygen saturation estimate, might significantly change during the monitoring of the individual by optical plethysmography, for example during sleep apnea.

From equations (6) and (7), the following can be obtained:

$$SpO_2 V_{blood} = V_{HbO_2}$$

$$(1 - SpO_2)V_{blood} = V_{Hb} \tag{8}$$

By substituting expression (8) into equation (5), the following can be obtained:

$$ln\left(\frac{I_l}{I_h}\right) = -d\left(\left(\varepsilon_{HbO_2} - \varepsilon_{other}\right)SpO_2\,\Delta V_{blood} + (\varepsilon_{Hb} - \varepsilon_{other})(1 - SpO_2)\Delta V_{blood}\right) \tag{9}$$

Two predetermined calibration coefficients $Q_1$ and $Q_2$, which are two constants, can be defined as follows:

$$Q_1 = \varepsilon_{HbO_2} - \varepsilon_{Hb}$$

$$Q_2 = \varepsilon_{Hb} - \varepsilon_{other}$$

After rewriting equation (9) in view of the two predetermined calibration coefficients $Q_1$ and $Q_2$, equation (10) can be obtained, wherein the left-hand side of equation (10) corresponds to an evaluation function, and wherein $Q_1 SpO_2 + Q_2$ corresponds to a compensation function which is a function of the oxygen saturation estimate:

$$ln\left(\frac{I_l}{I_h}\right) = -d\Delta V_{blood}(Q_1 SpO_2 + Q_2) \tag{10}$$

Equation (10) highlights a term on the left-hand side, referred to as the evaluation function, which shows a linear relationship with:

- the optical path length $d$;
- one or more changes in arterial blood volume $\Delta V_{blood}$, which is the parameter of interest; and
- a factor $(Q_1 SpO_2 + Q_2)$, which linearly depends on the oxygen saturation estimate.

Under the assumption of a constant volume fraction or concentration of the sum of oxygenated and deoxygenated hemoglobin in the arterial blood, $\Delta V_{blood}$ is a linear proxy for arterial blood volume fluctuations within the investigated volume, and hence corresponds to a measurement of peripheral arterial tone.

For a constant value of the oxygen saturation estimate, a change in arterial blood volume in the investigated volume between two points in time is thus evaluated when determining the logarithm of the ratio of the light intensities collected on the sensor when measuring with optical plethysmography at said two points in time.

$$ ln\left(\frac{I_l}{I_h}\right) \sim \Delta V_{blood} \, , if \, SpO_2 \, is \, constant \qquad (11) $$

As only a linear relationship between a measurable parameter and $\Delta V_{blood}$ is relevant to investigate relative changes in $\Delta V_{blood}$ rather than the exact $\Delta V_{blood}$ value, determination of $\Delta V_{blood}$ up to a constant factor is sufficient, as is performed by determining the evaluation function.

If the $SpO_2$ is not constant, the evaluation function will also change with changes in $SpO_2$. However, certain compensations methods can be used to compensate for the effect of changes in SpOz.

According to example embodiments, the function of the light intensities corresponds to a ratio of the light intensities.

According to equation (3), the evaluation function corresponds to the natural logarithm of a function of the light intensities. Alternatively, starting from equation (2), any other evaluation function defined as a function of the light intensities could be used, for example a linear approximation of the logarithm of a function of the light intensities, or for example a Taylor series approximation of a function of the light intensities, or for example a linear approximation of other base logarithms of a function of the light intensities. Alternatively, the evaluation function corresponds approximately to the ratio of the pulsatile waveform or AC component of the optical plethysmography signal and of the slowly varying baseline or DC component of the optical plethysmography signal, resulting in equation (13):

$$ ln\left(\frac{I_h}{I_l}\right) = ln\left(\frac{I_l}{I_l} + \frac{I_h - I_l}{I_l}\right) = ln\left(1 + \frac{I_h - I_l}{I_l}\right) \cong \frac{I_h - I_l}{I_l} \equiv \frac{AC_{ppg}}{DC_{ppg}} \qquad (13) $$

According to example embodiments, the function of the light intensities include a first measure corresponding to the light intensity measured by the sensor of the optical plethysmography setup at a first point in time and a second measure corresponding to the light intensity measured by the sensor of the optical plethysmography setup at a second point in time.

According to example embodiments, the function of the light intensities that corresponds to the ratio of the light intensities (102; 103) is a ratio of a first measure corresponding to the light intensity measured by the sensor of the optical plethysmography setup at a first point in time and a second measure corresponding to the light intensity measured by the sensor of the optical plethysmography setup at a second point in time.

According to example embodiments, the first point in time corresponds to the diastole in a first cardiac cycle and the second point in time corresponds to the systole in a second cardiac cycle different from the first cardiac cycle.

According to example embodiments, the first point in time corresponds to the systole in a first cardiac cycle and the second point in time corresponds to the diastole in a second cardiac cycle different from the first cardiac cycle.

According to example embodiments, the first point in time corresponds to the diastole in a cardiac cycle and the second point in time corresponds to the systole in the same cardiac cycle.

According to example embodiments, the first point in time corresponds to the systole in a cardiac cycle and the second point in time corresponds to the diastole in the same cardiac cycle.

According to example embodiments, the evaluation function corresponds to a logarithm of a ratio of the light intensities; and the evaluation function depends on one or more of the following:

- an optical path length;
- a function of the oxygen saturation estimate;
- the changes in arterial blood volume in the investigated volume.

According to example embodiments, at least one of the points in time corresponds to the diastole in a cardiac cycle of the individual and/or wherein at least one of the points in time corresponds to the systole in a cardiac cycle of the individual.

During systole, the volume of arterial blood in the investigated volume of the individual is maximum, resulting in the largest absorption and scattering of light of any point in time within a cardiac cycle, i.e. the period between two heart beats, since hemoglobin is one of the main absorbers and scatters of photons in the investigated volume, hence resulting in the lowest measured light intensity on the sensor of the optical plethysmography setup. Conversely, during diastole, the volume of arterial blood in the investigated volume of the individual is minimum, resulting in the lowest absorption and scattering of light of any point in time within a cardiac cycle and hence highest measured light intensity on the sensor of the optical plethysmography setup. At least one first point in time corresponds for example to the diastole in a first cardiac cycle and/or at least one second point in time corresponds for example to the systole in a second cardiac cycle different from the first cardiac cycle. Alternatively, at least one first point in time corresponds for example to the systole in a first cardiac cycle and/or at least one second point in time corresponds for example to the diastole in a second cardiac cycle different from the first cardiac cycle. Alternatively, at least one first point in time corresponds for example to the systole or to the diastole in a cardiac cycle and at least one second point in time corresponds to any point in time within the same cardiac cycle or within a different cardiac cycle.

According to example embodiments, the method further comprises the steps of:

- operating a light source configured to emit light at a wavelength;
- operating a sensor;
- collecting, by optical plethysmography and on the sensor, propagated light corresponding to light at the wavelength being transmitted or reflected when propagating in the investigated volume of the individual at the two or more points in time; and
- determining the light intensities of the propagated light on the sensor at the two or more points in time.

Optical plethysmography technology uses a simple and noninvasive setup probe or biosensor. The optical plethysmography biosensor non-invasively measures pulsatile arterial volume changes in the investigated volume, and thereby assesses PAT, by collecting the optical plethysmography signal. The light source is for example a LED or any other suitable light source which can be miniaturized to fit in the optical plethysmography biosensor. The wavelength is for example comprised in the red spectrum. Alternatively, the wavelength is comprised in the infra-red spectrum. A physical distance between the light sources and the sensor is for example a few millimeters, such as for example less than 3mm.

According to example embodiments, the method further comprises the step of:
detecting the occurrence at the individual under monitoring of the sleep-related event by determining a drop in value of the logarithm of the function of the light intensities below a predetermined threshold value.

According to a second example aspect, an apparatus according to claim 11 is disclosed.

The apparatus according to the present disclosure allows determining peripheral arterial tone in an accurate and robust manner. By determining a logarithm or a function approximation therefore of a function of the light intensities, the determination of changes in arterial blood volume in the investigated volume between two points in time with the apparatus according to the present disclosure is more accurate than by monitoring changes in amplitude of pulses in the optical plethysmography signal. Therefore, with the apparatus according to the present disclosure, it becomes possible to assess PAT of an individual more accurately and more robustly than with existing prior art solutions. In other words, the resulting evaluation of changes in arterial blood volume in the investigated volume through the determination of a logarithm or a function approximation thereof of a function of the light intensities therefore provides a more accurate and robust assessment of peripheral arterial tone of the individual.

According to example embodiments, a system is provided, wherein the system comprises an apparatus according to a second example aspect of the invention, and further comprises:

- a light source configured to emit light; and
- the sensor configured to collect by optical plethysmography propagated light corresponding to the light being transmitted or reflected when propagating in the investigated volume of the individual at the two or more points in time; and further configured to determine the light intensities of the propagated light at the two or more points in time.

The sensor collects propagated light by optical plethysmography, wherein the propagated light corresponds to the light being transmitted or reflected when propagating in the investigated volume of the individual, such as for example a distal end of a digit of the individual, at the two or more points in time. The system further optionally comprises a wireless transmitter comprising a wireless communication interface, wherein the wireless transmitter is configured to transmit the determined peripheral arterial tone wirelessly for further processing by the apparatus. The wireless communication interface is preferably a low power communication interface, e.g. a Bluetooth Low Energy, BLE, wireless interface.

According to a third example aspect, a computer program product comprising computer-executable instructions for causing a system to perform the method according to a first example aspect is provided.

According to a fourth example aspect, a computer readable storage medium is provided, wherein the computer readable storage medium comprises computer-executable instructions for performing the method according to a first example aspect when the program is run on a computer.

According to a fifth example aspect, a use of a logarithm or a function approximation thereof for assessing peripheral arterial tone, PAT, of an individual monitored by optical plethysmography, is provided, wherein assessing peripheral arterial tone comprises:

- obtaining:

  ∘ an optical plethysmography signal measured at an investigated volume of the individual; and
  ∘ light intensities acquired by optical plethysmography at two or more points in time along the optical plethysmography signal; and

- determining changes in arterial blood volume in the investigated volume between the two or more points in time by determining a logarithm or a function approximation thereof of a function of the light intensities, thereby assessing PAT of the individual.

## Brief Description of the Drawings

Some example embodiments will now be described with reference to the accompanying drawings.

Fig. 1 depicts an example embodiment of an apparatus according to the present disclosure.

Fig. 2 depicts an example embodiment of a system according to the present disclosure comprising an apparatus according to the present disclosure.

Fig. 3 depicts an example embodiment of a system according to the present disclosure comprising an apparatus according to the present disclosure.

Fig. 4 depicts an example embodiment of a comparison of a PAT measurement of an individual assessed by an apparatus according to the present disclosure and assessed without determining a logarithm of a function of the light intensities.

Fig. 5 depicts an example embodiment of a computer-implemented method according to the present disclosure.

Fig. 6 shows an example embodiment of a suitable computing system for performing one or several steps in embodiments of the invention.

## Detailed Description of Embodiment(s)

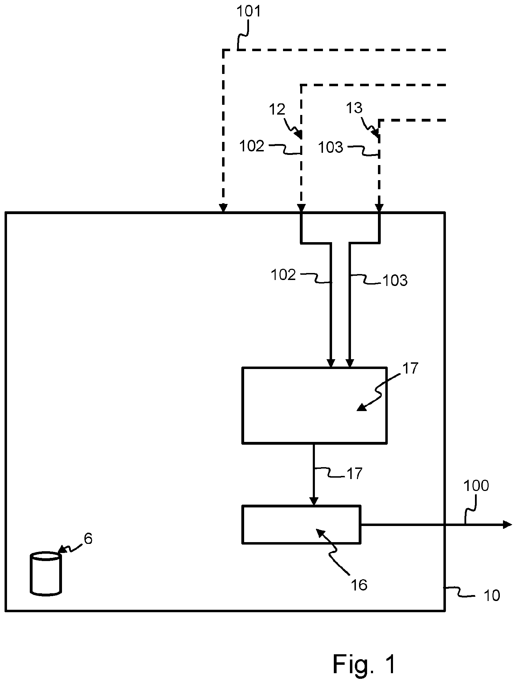

Fig. 1 illustrates an example embodiment of an apparatus 10 according to the present disclosure. The apparatus 10 comprises at least one memory 6, at least one processor, wherein the memory 6 includes computer program code configured to, with the at least one processor, cause the apparatus 10 to perform the following:

- obtain:

  ∘ an optical plethysmography signal 101 measured at an investigated volume of an individual by a sensor of an optical plethysmography setup; and
  ∘ light intensities 102;103 acquired by optical plethysmography at two or more points in time 12;13 along the optical plethysmography signal 101, wherein the light intensity 102 is respectively acquired at the point in time 12 and wherein the light intensity 103 is respectively acquired at the point in time 13;

- determining changes in arterial blood volume 16 in the investigated volume between the two or more points in time 12;13 by determining a logarithm 17 or a function approximation thereof 17 of a function of the light intensities 102;103, thereby assessing PAT 100 of the individual; and

- determining a drop in the PAT 100 indicative for a vasoconstriction in the investigated volume 11, thereby detecting occurrence of a sleep-related event at the individual 1.

The apparatus 10 obtains the optical plethysmography signal 101 and/or the light intensities 102;103 from an external device. According to an alternative embodiment, the apparatus 10 obtains the optical plethysmography signal 101 and/or the light intensities 102;103 from the memory 6. According to a further alternative embodiment, the apparatus 10 the optical plethysmography signal 101 and/or the light intensities 102;103 from the memory 6 and/or from an external device. Optionally, at least one point in time 12 corresponds to the diastole of a cardiac cycle of the individual and/or at least one point in time 13 corresponds to the systole of a cardiac cycle of the individual. The apparatus 10 is configured to determine an evaluation function 17 which corresponds to a logarithm 17 or a function approximation thereof 17 of a function of the light intensities 102; 103. The evaluation function 17 depends on one or more of the following: an optical path length, a function of the oxygen saturation estimate, a change in arterial blood volume in the investigated volume.

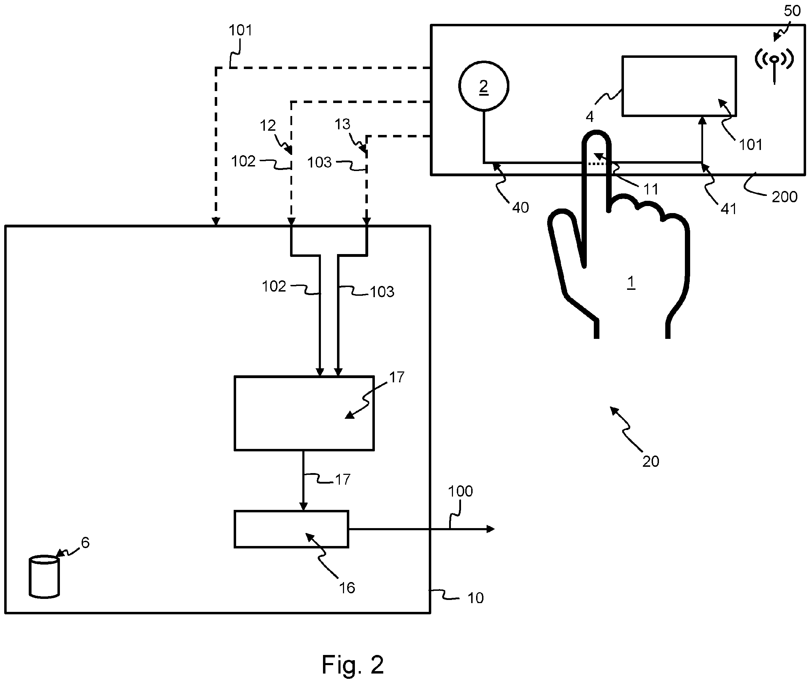

Fig. 2 illustrates an example embodiment of a system 20 according to the present disclosure. Components having identical reference numbers to numbers on Fig. 1 perform the same function. The system 20 of Fig. 2 comprises an apparatus 10 according to the present disclosure. Optionally, the system 20 further comprises a light source 2 and a sensor 4. A light source 2 is configured to emit light 40. The apparatus 10 is configured to:

- obtain:

  ◦ an optical plethysmography signal 101 measured at an investigated volume 11 of an individual by a sensor of an optical plethysmography setup; and
  ◦ light intensities 102;103 acquired by optical plethysmography at two or more points in time 12;13 along the optical plethysmography signal 101, wherein the light intensity 102 is respectively acquired at the point in time 12 and wherein the light intensity 103 is respectively acquired at the point in time 13;

    - determining changes in arterial blood volume 16 in the investigated volume 11 between the two or more points in time 12;13 by determining a logarithm 17 or a function approximation thereof 17 of a function of the light intensities 102;103, thereby assessing PAT 100 of the individual 1; and
    - determining a drop in the PAT 100 indicative for a vasoconstriction in the investigated volume 11, thereby detecting occurrence of a sleep-related event at the individual 1.

The apparatus 10 obtains the optical plethysmography signal 101 and/or the light intensities 102;103 from an external device from an external device 200 comprising at least one light source 2 and/or the sensor 4. For example, the external device 200 determines the arterial blood volume pulse in the investigated volume 11 of the individual 1. The external device 200 comprises a battery for powering the different electrical components 2;4. The light source 2 is configured to emit light, i.e. to transmit the light 40 into the investigated volume 11 of the individual attached to the external device 200, for example into the finger 11 of the individual 1 as illustrated, more particularly to a distal end 11 of a finger of the individual. The external device 200 further comprises control circuitry for controlling the light source 2, i.e., for enabling or disabling the light source 2 and for receiving the measurements for example of the light intensities from the sensor 4. Control circuitry may further comprise a memory component for temporarily storing the obtained measurements. Control circuitry is further coupled to a wireless interfacing circuitry 50 and configured to forward the measurements to the wireless interfacing circuitry 50. Wireless interface 50 may support a short range and/or low power wireless communication protocol for efficient transmission of the measurements to a receiving part of the system. Wireless interface 50 may for example operate according to the Bluetooth Low Energy, BLE, protocol as defined by the Bluetooth Special Interest Group or according to a Near Field Communication, NFC, protocol. Operation by such protocols together with forwarding of the raw optical plethysmography signal 101 allows miniaturization of the external device 200 such that it fits on a finger or a nostril and allows operation during multiple nights According to an alternative embodiment, the apparatus 10 obtains the optical plethysmography signal 101 and/or the light intensities 102;103 from the memory 6. According to a further alternative embodiment, the apparatus 10 the optical plethysmography signal 101 and/or the light intensities 102;103 from the memory 6 and/or from an external device. Optionally, at least one point in time 12 corresponds to the diastole of a cardiac cycle of the individual and/or at least one point in time 13 corresponds to the systole of a cardiac cycle of the individual. The apparatus 10 is configured to determine an evaluation function 17 which is a function of the light intensities 102;103. The external device 200 collects, by optical plethysmography on the sensor 4, propagated light 41 corresponding to light 40 emitted by the light source 2, wherein the light 40 is transmitted or reflected by the investigated volume 11 when propagating in the distal end of the digit of the individual 1 at the two or more points in time 12;13 along the optical plethysmography signal 101. In other words, the external device 200 collects, by optical plethysmography on the sensor 4, a light intensity 102 corresponding to propagated light 41 corresponding to light 40 emitted by the light source 2, wherein the light 40 is transmitted or reflected by the investigated volume 11 when propagating in the distal end of the digit of the individual 1 and is collected on the sensor 4 at a first point in time 12; and the external device 200 collects, by optical plethysmography on the sensor 4, a light intensity 103 corresponding to propagated light 41 corresponding to light 40 emitted by the same light source 2, wherein the light 40 is transmitted or reflected by the investigated volume 11 when propagating in the distal end of the digit of the individual 1 and is collected on the sensor 4 at a second point in time 13. The apparatus then determines an evaluation function 17. The evaluation function 17 for example corresponds to a logarithm of a function of the light intensities 102;103. The evaluation function 17 for example corresponds to a logarithm of a ratio of the light intensities 102;103. The evaluation function 17 depends on one or more of the following: an optical path length, a function of the oxygen saturation estimate, a change in arterial blood volume in the investigated volume.

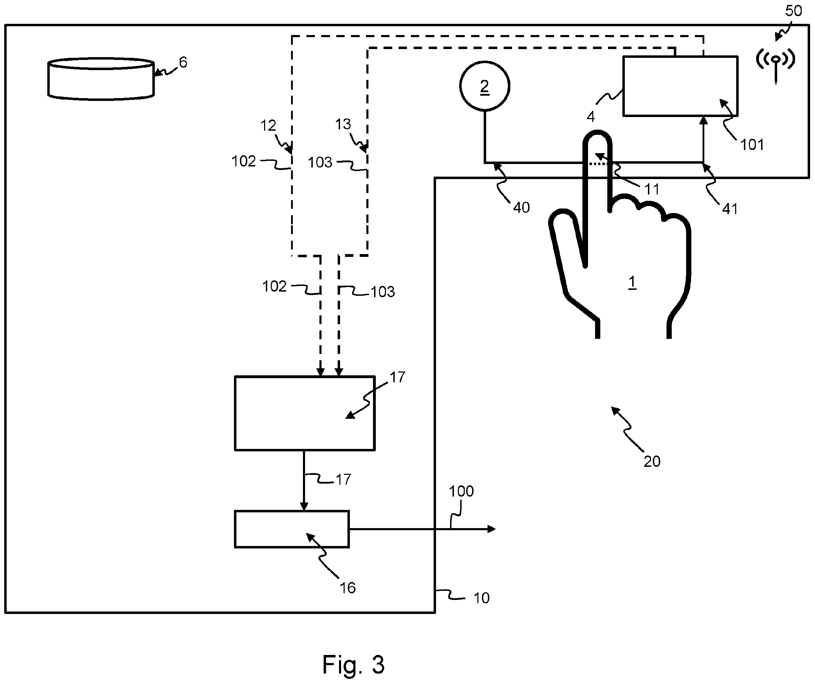

Fig. 3 illustrates an example embodiment of a system 20 according to the present disclosure. Components having identical reference numbers to numbers on Fig. 1 or Fig. 2 perform the same function. The system 20 of Fig. 3 comprises an apparatus 10 according to the present disclosure. Optionally, the system 20 further comprises at least one light source 2 and a sensor 4, comprised in the apparatus 10. A light source 2 is configured to emit light 40. The apparatus 10 is configured to:

- obtain:

  ◦ an optical plethysmography signal 101 measured at an investigated volume 11 of an individual by a sensor of an optical plethysmography setup; and
  ◦ light intensities 102;103 acquired by optical plethysmography at two or more points in time 12;13 along the optical plethysmography signal 101, wherein the light intensity 102 is respectively acquired at the point in time 12 and wherein the light intensity 103 is respectively acquired at the point in time 13;

- determining changes in arterial blood volume 16 in the investigated volume 11 between the two or more points in time 12;13 by determining a logarithm 17 or a function approximation thereof 17 of a function of the light intensities 102;103, thereby assessing PAT 100 of the individual 1; and
- determining a drop in the PAT 100 indicative for a vasoconstriction in the investigated volume 11, thereby detecting occurrence of a sleep-related event at the individual 1.

The apparatus 10 obtains the optical plethysmography signal 101 and/or the light intensities 102;103 from the light source 2 and/or the sensor 4. For example, the apparatus 10 determines the arterial blood volume pulse in the investigated volume 11 of the individual 1. The apparatus 10 comprises a battery for powering the different electrical components 2;3;4. The light source 2 is configured to emit light, i.e. to transmit the light 40 into the investigated volume 11 of the individual attached to the apparatus 10, for example into the finger 11 of the individual 1 as illustrated, more particularly to a distal end 11 of a finger of the individual as illustrated. The apparatus 10 further comprises control circuitry for controlling the light source 2, i.e., for enabling or disabling the light source 2 and for receiving the measured arterial blood volume pulse values from the sensor 4. Control circuitry may further comprise a memory component for temporarily storing the obtained measurements. Control circuitry is further coupled to a wireless interfacing circuitry 50 and configured to forward the measurements to the wireless interfacing circuitry 50. Wireless interface 50 may support a short range and/or low power wireless communication protocol for efficient transmission of the measurements to a receiving part of the system. Wireless interface 50 may for example operate according to the Bluetooth Low Energy, BLE, protocol as defined by the Bluetooth Special Interest Group or according to a Near Field Communication, NFC, protocol. Operation by such protocols together with forwarding of the raw optical plethysmography signal 101 allows miniaturization of the apparatus 10 such that it fits on a finger or a nostril and allows operation during multiple nights. According to an alternative embodiment, the apparatus 10 obtains one or more of the optical plethysmography signal 101, the light intensities 102;103 from the memory 6. According to a further alternative embodiment, the apparatus 10 obtains one or more of the optical plethysmography signal 101, the light intensities 102;103 from the light source 2 and/or the sensor 4 and/or from the memory 6. Optionally, at least one point in time 12 corresponds to the diastole of a cardiac cycle of the individual and/or at least one point in time 13 corresponds to the systole of a cardiac cycle of the individual. The apparatus 10 is configured to determine an evaluation function 17 which is a function of the light intensities 102;103. The apparatus 10 collects, by optical plethysmography on the sensor 4, propagated light 41 corresponding to light 40 emitted by the light source 2, wherein the light 40 is transmitted or reflected by the investigated volume 11 when propagating in the distal end of the digit of the individual at the two or more points in time 12;13 along the optical plethysmography signal 101. In other words, the apparatus 10 collects, by optical plethysmography on the sensor 4, a light intensity 102 corresponding to propagated light 41 corresponding to light 40 emitted by the light source 2, wherein the light 40 is transmitted or reflected by the investigated volume 11 when propagating in the distal end of the digit of the individual 1 and is collected on the sensor 4 at a first point in time 12; and the apparatus 10 collects, by optical plethysmography on the sensor 4, a light intensity 103 corresponding to propagated light 41 corresponding to light 40 emitted by the same light source 2, wherein the light 40 is transmitted or reflected by the investigated volume 11 when propagating in the distal end of the digit of the individual 1 and is collected on the sensor 4 at a second point in time 13. The apparatus then determines an evaluation function 17. The evaluation function 17 for example corresponds to a logarithm of a function of the light intensities 102;103. The evaluation function 17 for example corresponds to a logarithm of a ratio of the light intensities 102;103. The evaluation function 17 depends on one or more of the following: an optical path length, a function of the oxygen saturation estimate, a change in arterial blood volume in the investigated volume.

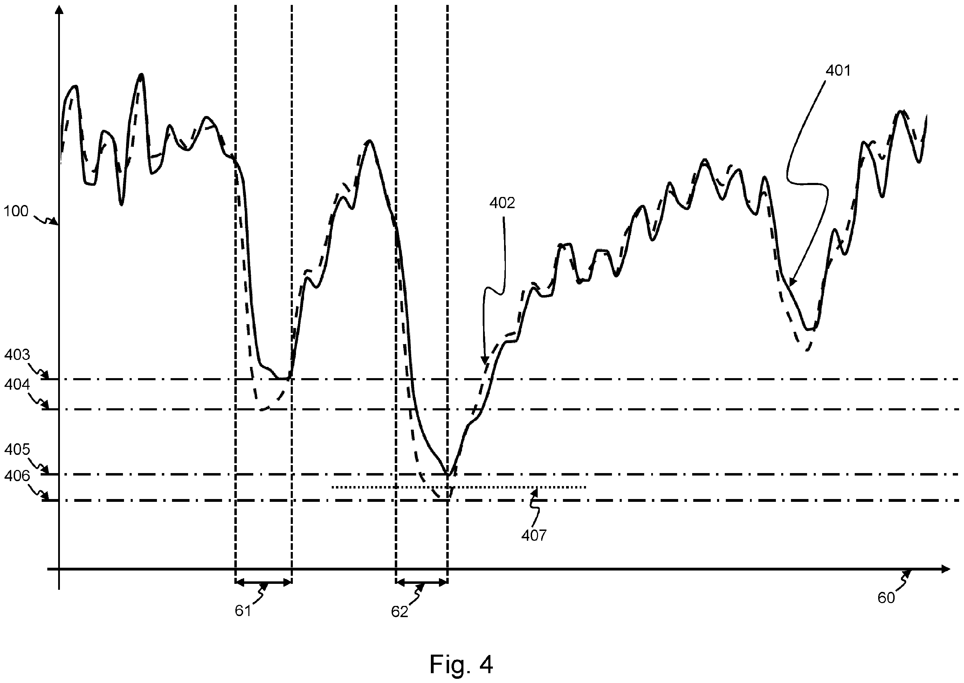

Fig. 4 illustrates an example comparison between PPG-amplitude-based peripheral arterial tone 401 both in function of time 60 of an individual, wherein the PPG-amplitude-based peripheral arterial tone 401 is assessed by considering changes in amplitude of pulses in an optical plethysmography signal as described in the prior art, and evaluation-function-based peripheral arterial tone 402 in function of time 60 of the same individual, wherein the evaluation-function-based peripheral arterial tone 402 is determined by the computer-implemented method according to the present disclosure, or by the apparatus according to the present disclosure, i.e. wherein the evaluation-function-based peripheral arterial tone 402 is assessed by determining a logarithm of a ratio of light intensities measured from an optical plethysmography signal. For clarity reasons, the evaluation-function-based peripheral arterial tone 402 is plotted by calculating

$$ln\left(\frac{I_h}{I_l}\right)$$

. According to an alternative embodiment, the evaluation-function-based peripheral arterial tone 402 is plotted by calculating $ln\left(\frac{I_l}{I_h}\right)$ . As can be seen on Fig. 4, the PPG-amplitude-based peripheral arterial tone 401 and the evaluation-function-based peripheral arterial tone 402 evolve in a similar manner as a function of time 60 at a pre-vasoconstriction-event baseline value. But during the time periods 61 and 62, i.e. during occurrence of an event such as for example a sleep-related event, it can be seen on Fig. 4 that the PPG-amplitude-based peripheral arterial tone 401 and the evaluation-function-based peripheral arterial tone 402 evolve in a similar manner but do not overlap anymore in the corresponding time periods 61 and 62. Indeed, in period 61, the PPG-amplitude-based peripheral arterial tone 401 drops to a level 403 which value is higher than the one of the level 404 to which the evaluation-function-based peripheral arterial tone 402 drops. Similarly, in period 62, the PPG-amplitude-based peripheral arterial tone 401 drops to a level 405 which value is higher than the one of the level 406 to which the evaluation-function-based peripheral arterial tone 402 drops. It can be seen on Fig. 4 that assessing peripheral arterial tone 100 by determining a logarithm of a function of light intensities acquired by optical plethysmography allows to detect the event occurring more accurately. Indeed, a drop in peripheral arterial tone 100 is indicative for a vasoconstriction of the arteries and the arterioles in the investigated volume under monitoring. This vasoconstriction can be related to the occurrence of an event at the individual under monitoring such as for example a sleep-related event, such as for example sleep apnea. As it can be seen on Fig. 4, the drop in the PPG-amplitude-based peripheral arterial tone 401 between the pre-vasoconstriction-event baseline value and the lowest point of the PPG-amplitude-based peripheral arterial tone 401 is smaller than the drop in the evaluation-function-based peripheral arterial tone 402 between the pre-vasoconstriction-event baseline value and the lowest point of the evaluation-function-based peripheral arterial tone 402. For example, a predetermined threshold value 407 for peripheral arterial tone 100 can be used to detect whether an event is occurring at the individual under monitoring such as for example a sleep-related event, such as for example sleep apnea: when the peripheral arterial tone 100 is above this predetermined threshold value 407, no event is detected, but when the peripheral arterial tone 100 is below this predetermined threshold value 407, an event is detected. The drop in the PPG-amplitude-based peripheral arterial tone 401 between the pre-vasoconstriction-event baseline value and the lowest point of the PPG-amplitude-based peripheral arterial tone 401 is such that the PPG-amplitude-based peripheral arterial tone 401 stays above the predetermined threshold value 407, which results in the absence of detection of an event occurring at the individual under monitoring such as for example a sleep-related event, such as for example sleep apnea. On the other hand, the drop in the evaluation-function-based peripheral arterial tone 402 between the pre-vasoconstriction-event baseline value and the lowest point of the evaluation-function-based peripheral arterial tone 402 is such that the evaluation-function-based peripheral arterial tone 402 drops below the predetermined threshold value 407, which results in the detection of an event occurring at the individual under monitoring such as for example a sleep-related event, such as for example sleep apnea. It can therefore be seen that assessing peripheral arterial tone 100 by determining a logarithm of a function of light intensities acquired by optical plethysmography allows to more accurately and more robustly detect the occurrence of an event occurring at the individual under monitoring such as for example a sleep-related event, such as for example sleep apnea.

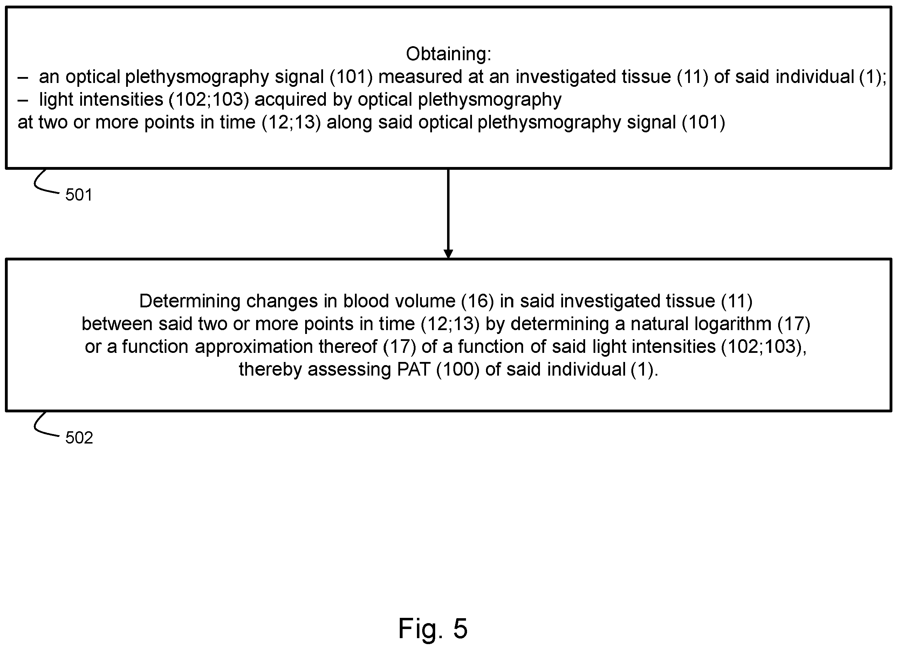

Fig. 5 illustrates an example embodiment of a computer-implemented method for assessing peripheral arterial tone, PAT, of an individual monitored by optical plethysmography, wherein said method comprises the steps of:

- in a first step 501, obtaining:

○ an optical plethysmography signal 101 measured at an investigated volume 11 of the individual 1 by a sensor of an optical plethysmography setup; and
○ light intensities 102;103 acquired by optical plethysmography at two or more points in time 12;13 along the optical plethysmography signal 101; and

- in a second step 502 consecutive to the first step 501, determining changes in arterial blood volume 16 in the investigated volume 11 between the two or more points in time 12;13 by determining a logarithm 17 or a function approximation thereof 17 of a function of the light intensities 102;103, thereby assessing PAT 100 of the individual 1; and determining a drop in the PAT 100 indicative for a vasoconstriction in the investigated volume 11, thereby detecting occurrence of a sleep-related event at the individual 1.

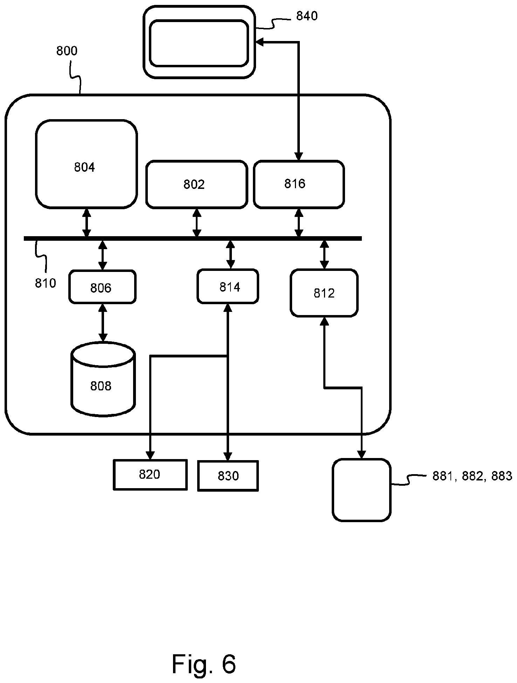

Fig. 6 shows a suitable computing system 800 enabling to implement embodiments of the system. Computing system 800 may in general be formed as a suitable general-purpose computer and comprise a bus 810, a processor 802, a local memory 804, one or more optional input interfaces 814, one or more optional output interfaces 816, a communication interface 812, a storage element interface 806, and one or more storage elements 808. Bus 810 may comprise one or more conductors that permit communication among the components of the computing system 800. Processor 802 may include any type of conventional processor or microprocessor that interprets and executes programming instructions. Local memory 804 may include a random-access memory (RAM) or another type of dynamic storage device that stores information and instructions for execution by processor 802 and/or a read only memory (ROM) or another type of static storage device that stores static information and instructions for use by processor 802. Input interface 814 may comprise one or more conventional mechanisms that permit an operator or user to input information to the computing device 800, such as a keyboard 820, a mouse 830, a pen, voice recognition and/or biometric mechanisms, a camera, etc. Output interface 816 may comprise one or more conventional mechanisms that output information to the operator or user, such as a display 840, etc. Communication interface 812 may comprise any transceiver-like mechanism such as for example one or more Ethernet interfaces that enables computing system 800 to communicate with other devices and/or systems, for example with other computing devices 881, 882, 883. The communication interface 812 of computing system 800 may be connected to such another computing system by means of a local area network (LAN) or a wide area network (WAN) such as for example the internet. Storage element interface 806 may comprise a storage interface such as for example a Serial Advanced Technology Attachment (SATA) interface or a Small Computer System Interface (SCSI) for connecting bus 810 to one or more storage elements 808, such as one or more local disks, for example SATA disk drives, and control the reading and writing of data to and/or from these storage elements 808. Although the storage element(s) 808 above is/are described as a local disk, in general any other suitable computer-readable media such as a removable magnetic disk, optical storage media such as a CD or DVD, -ROM disk, solid state drives, flash memory cards, ... could be used. Computing system 800 could thus correspond to the apparatus 10 in the embodiment illustrated by Figs. 1 or 2 or 3.

As used in this application, the term "circuitry" may refer to one or more or all of the following:

(a) hardware-only circuit implementations such as implementations in only analog and/or digital circuitry and
(b) combinations of hardware circuits and software, such as (as applicable):

(i) a combination of analog and/or digital hardware circuit(s) with software/firmware and

(ii) any portions of hardware processor(s) with software (including digital signal processor(s)), software, and memory(ies) that work together to cause an apparatus, such as a mobile phone or server, to perform various functions) and

(c) hardware circuit(s) and/or processor(s), such as microprocessor(s) or a portion of a microprocessor(s), that requires software (e.g. firmware) for operation, but the software may not be present when it is not needed for operation.

This definition of circuitry applies to all uses of this term in this application, including in any claims. As a further example, as used in this application, the term circuitry also covers an implementation of merely a hardware circuit or processor (or multiple processors) or portion of a hardware circuit or processor and its (or their) accompanying software and/or firmware. The term circuitry also covers, for example and if applicable to the particular claim element, a baseband integrated circuit or processor integrated circuit for a mobile device or a similar integrated circuit in a server, a cellular network device, or other computing or network device.

Although the present invention has been illustrated by reference to specific embodiments, it will be apparent to those skilled in the art that the invention is not limited to the details of the foregoing illustrative embodiments, and that the present invention may be embodied with various changes and modifications without departing from the scope thereof.

The present embodiments are therefore to be considered in all respects as illustrative and not restrictive, the scope of the invention being indicated by the appended claims rather than by the foregoing description, and all changes which come within the scope of the claims are therefore intended to be embraced therein.

It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above.

## Claims

1. A computer-implemented method for assessing peripheral arterial tone (100), PAT, of an individual (1) monitored by optical plethysmography, wherein said method comprises:

   - obtaining:

      ◦ an optical plethysmography signal (101) measured at an investigated volume (11) of said individual (1) by a sensor of an optical plethysmography setup; and
      ◦ light intensities (102;103) acquired by optical plethysmography at two or more points in time (12;13) along said optical plethysmography signal (101); and

   - determining changes in arterial blood volume (16) in said investigated volume (11) between said two or more points in time (12;13) by determining an evaluation function that determines a logarithm (17), or a function approximation thereof (17), of a ratio of said light intensities (102;103), thereby assessing PAT (100) of said individual (1); said two or more points in time including at least one first point in time corresponding to a systole or to a diastole in a cardiac cycle and at least one second point in time corresponding to any point in time within the same cardiac cycle or within a different cardiac cycle; and
   - determining a drop in said PAT (100) indicative for a vasoconstriction in said investigated volume (11), by determining a drop in value of the evaluation function that determines the logarithm (17), or the function approximation thereof, of the ratio of said light intensities, below a predetermined threshold value.

2. The method according to claim 1, wherein the ratio of said light intensities include a first measure corresponding to the light intensity measured by the sensor of the optical plethysmography setup at a first point in time and a second measure corresponding to the light intensity measured by the sensor of the optical plethysmography setup at a second point in time.

3. The method according to claim 1, wherein said ratio of said light intensities (102;103) is a ratio of a first measure corresponding to the light intensity measured by the sensor of the optical plethysmography setup at a first point in time and a second measure corresponding to the light intensity measured by the sensor of the optical plethysmography setup at a second point in time.

4. The method according to claim 3, wherein the first point in time corresponds to the diastole in a first cardiac cycle and the second point in time corresponds to the systole in a second cardiac cycle different from the first cardiac cycle.

5. The method according to claim 3, wherein the first point in time corresponds to the systole in a first cardiac cycle and the second point in time corresponds to the diastole in a second cardiac cycle different from the first cardiac cycle.

6. The method according to claim 3, wherein the first point in time corresponds to the diastole in a cardiac cycle and the second point in time corresponds to the systole in a cardiac cycle.

7. The method according to claim 3, wherein the first point in time corresponds to the systole in a cardiac cycle and the second point in time corresponds to the diastole in a cardiac cycle.

8. The method according to any of claims 1 to 3, wherein at least one (12) of said points in time (12;13) corresponds to the diastole in a cardiac cycle of said individual (1) and/or wherein at least one (13) of said points in time (12;13) corresponds to the systole in a cardiac cycle of said individual (1).

9. The method according to any of the preceding claims, wherein said method further comprises the steps of:

- operating a light source (2) configured to emit light (40) at a wavelength;
- operating a sensor (4);
- collecting, by optical plethysmography and on said sensor (4), propagated light (41) corresponding to light (40) at said wavelength being transmitted or reflected when propagating in said investigated volume (11) of said individual (1) at said two or more points in time (12;13); and
- determining said light intensities (102;103) of said propagated light on said sensor (4) at said two or more points in time (12;13).

10. A computer readable storage medium comprising computer-executable instructions for performing the method of any one of claims 1 to 9 when the program is run on a computer.

11. An apparatus (10) comprising at least one processor and at least one memory (6) including computer program code, the at least one memory (6) and computer program code configured to, with the at least one processor, cause the apparatus (10) to perform the method of any one of claims 1 to 9.

12. The apparatus according to claim 11, wherein, when said PAT (100) is below said predetermined threshold value, a sleep-related event is detected, and wherein, when said PAT (100) is above said predetermined threshold value, no sleep-related event is detected.

13. The apparatus according to claim 12, wherein said sleep-related event is sleep apnea.

14. A system (20) comprising the apparatus according to any one of claims 11 to 13, and further comprising:

- a light source (2;3) configured to emit light; and
- the sensor (4) configured to collect by optical plethysmography propagated light corresponding to said light being transmitted or reflected when propagating in said investigated volume (11) of said individual (1) at said two or more points in time (12;13); and further configured to determine said light intensities (102; 103) of said propagated light at said two or more points in time (12;13).

**Patentansprüche**

1. Computerimplementiertes Verfahren zur Beurteilung des peripheren arteriellen Tonus, PAT, (100) eines Individuums (1), der durch optische Plethysmographie überwacht wird, wobei das Verfahren umfasst:

- Erhalten:

  - eines optischen Plethysmographiesignals (101), das an einem untersuchten Volumen (11) des Individuums (1) durch einen Sensor einer Anordnung für optische Plethysmographie gemessen wird; und
  - von Lichtintensitäten (102; 103), die durch optische Plethysmographie zu zwei oder mehr Zeitpunkten (12; 13) entlang des optischen Plethysmographiesignals (101) erfasst werden; und

- Bestimmen von Änderungen des arteriellen Blutvolumens (16) im untersuchten Volumen (11) zwischen den zwei oder mehr Zeitpunkten (12; 13) durch Bestimmen einer Bewertungsfunktion, die einen Logarithmus (17) oder eine Funktionsnäherung davon (17) eines Verhältnisses der Lichtintensitäten (102; 103) bestimmt, um dadurch den PAT (100) des Individuums (1) zu beurteilen; wobei die zwei oder mehr Zeitpunkte mindestens einen ersten Zeitpunkt, der einer Systole oder einer Diastole in einem Herzzyklus entspricht, und mindestens einen zweiten Zeitpunkt umfassen, der einem beliebigen Zeitpunkt innerhalb desselben Herzzyklus oder innerhalb eines anderen Herzzyklus entspricht; und
- Bestimmen eines Abfalls des PAT (100), der auf eine Vasokonstriktion im untersuchten Volumen (11) hinweist, durch Bestimmen eines Abfalls des Wertes der Bewertungsfunktion, die den Logarithmus (17) oder die Funktionsnäherung davon des Verhältnisses der Lichtintensitäten bestimmt, unter einen vorgegebenen Schwellenwert.

2. Verfahren nach Anspruch 1, wobei das Verhältnis der Lichtintensitäten ein erstes Maß, das der vom Sensor der Anordnung für optische Plethysmographie zu einem ersten Zeitpunkt gemessenen Lichtintensität entspricht, und ein zweites Maß umfasst, das der vom Sensor der Anordnung für optische Plethysmographie zu einem zweiten Zeitpunkt gemessenen Lichtintensität entspricht.

3. Verfahren nach Anspruch 1, wobei das Verhältnis der Lichtintensitäten (102; 103) ein Verhältnis eines ersten Maßes, das der vom Sensor der Anordnung für optische Plethysmographie zu einem ersten Zeitpunkt gemessenen Lichtintensität entspricht, und eines zweiten Maßes ist, das der vom Sensor der Anordnung für optische Plethysmographie zu einem zweiten Zeitpunkt gemessenen Lichtintensität entspricht.

4. Verfahren nach Anspruch 3, wobei der erste Zeitpunkt der Diastole in einem ersten Herzzyklus entspricht und der zweite Zeitpunkt der Systole in einem vom ersten Herzzyklus verschiedenen zweiten Herzzyklus entspricht.

5. Verfahren nach Anspruch 3, wobei der erste Zeitpunkt der Systole in einem ersten Herzzyklus entspricht und der zweite Zeitpunkt der Diastole in einem vom ersten Herzzyklus verschiedenen zweiten Herzzyklus entspricht.

6. Verfahren nach Anspruch 3, wobei der erste Zeitpunkt der Diastole in einem Herzzyklus entspricht und der zweite Zeitpunkt der Systole in einem Herzzyklus entspricht.

7. Verfahren nach Anspruch 3, wobei der erste Zeitpunkt der Systole in einem Herzzyklus entspricht und der zweite Zeitpunkt der Diastole in einem Herzzyklus entspricht.

8. Verfahren nach einem der Ansprüche 1 bis 3, wobei mindestens einer (12) der Zeitpunkte (12; 13) der Diastole in einem Herzzyklus des Individuums (1) entspricht und/oder wobei mindestens einer (13) der Zeitpunkte (12; 13) der Systole in einem Herzzyklus des Individuums (1) entspricht.

9. Verfahren nach einem der vorhergehenden Ansprüche, wobei das Verfahren ferner die folgenden Schritte umfasst:

   - Betreiben einer Lichtquelle (2), die zum Emittieren von Licht (40) mit einer Wellenlänge ausgelegt ist;
   - Betreiben eines Sensors (4);
   - Erfassen, durch optische Plethysmographie und an dem Sensor (4), von sich ausbreitendem Licht (41), das dem Licht (40) mit der Wellenlänge entspricht, das bei der Ausbreitung im untersuchten Volumen (11) des Individuums (1) zu den zwei oder mehr Zeitpunkten (12; 13) durchgelassen oder reflektiert wird; und
   - Bestimmen der Lichtintensitäten (102; 103) des sich ausbreitenden Lichts am Sensor (4) zu den zwei oder mehr Zeitpunkten (12; 13).

10. Computerlesbares Speichermedium, umfassend computerausführbare Anweisungen zum Durchführen des Verfahrens nach einem der Ansprüche 1 bis 9 bei Ausführung des Programms auf einem Computer.

11. Vorrichtung (10), umfassend mindestens einen Prozessor und mindestens einen Speicher (6), der Computerprogrammcode umfasst, wobei der mindestens eine Speicher (6) und der Computerprogrammcode so ausgelegt sind, dass sie mit dem mindesten einen Prozessor die Vorrichtung (10) zum Durchführen des Verfahrens nach einem der Ansprüche 1 bis 9 veranlassen.

12. Vorrichtung nach Anspruch 11, wobei, wenn der PAT (100) unter dem vorgegebenen Schwellenwert liegt, ein schlafbezogenes Ereignis erkannt wird und, wenn der PAT (100) über dem vorgegebenen Schwellenwert liegt, kein schlafbezogenes Ereignis erkannt wird.

13. Vorrichtung nach Anspruch 12, wobei das schlafbezogene Ereignis Schlafapnoe ist.

14. System (20), umfassend die Vorrichtung nach einem der Ansprüche 11 bis 13 und ferner umfassend:

   - eine Lichtquelle (2; 3), die zum Emittieren von Licht ausgelegt ist; und
   - den Sensor (4), der so ausgelegt ist, dass er sich ausbreitendes Licht, das dem Licht entspricht, das bei Ausbreitung im untersuchten Volumen (11) des Individuums (1) zu den zwei oder mehr Zeitpunkten (12; 13) durchgelassen oder reflektiert wird, durch optische Plethysmographie erfasst; und ferner so ausgelegt ist, dass er die Lichtintensitäten (102; 103) des sich ausbreitenden Lichts zu den zwei oder mehr Zeitpunkten (12; 13) bestimmt.

**Revendications**

1. Procédé mis en oeuvre par ordinateur pour évaluer le tonus artériel périphérique (100), PAT, d'un individu (1) surveillé par pléthysmographie optique, dans lequel ledit procédé comprend :

   - l'obtention :

      - d'un signal de pléthysmographie optique (101) mesuré au niveau d'un volume étudié (11) dudit individu (1) par un capteur d'un dispositif de pléthysmographie optique ; et
      - d'intensités lumineuses (102 ; 103) acquises par pléthysmographie optique à deux instants ou plus (12 ; 13) le long dudit signal de pléthysmographie optique (101) ; et

      - la détermination de changements du volume sanguin artériel (16) dans ledit volume étudié (11) entre lesdits deux instants ou plus (12 ; 13) en déterminant une fonction d'évaluation qui détermine un logarithme (17), ou une approximation de fonction de celui-ci (17), d'un rapport desdites intensités lumineuses (102 ; 103), évaluant ainsi le PAT (100) dudit individu (1) ; lesdits deux instants ou plus incluant au moins un premier instant correspondant à une systole ou à une diastole dans un cycle cardiaque et au moins un second instant correspondant à n'importe quel instant dans le même cycle cardiaque ou dans un cycle cardiaque différent ; et
      - la détermination d'une baisse dudit PAT (100) indicative d'une vasoconstriction dans ledit volume étudié (11), en déterminant une baisse de la valeur de la fonction d'évaluation qui détermine le logarithme (17), ou l'approximation de fonction de celui-ci, du rapport desdites intensités lumineuses, en dessous d'une valeur seuil prédéterminée.

2. Procédé selon la revendication 1, dans lequel le rapport desdites intensités lumineuses inclut une première mesure correspondant à l'intensité lumineuse mesurée par le capteur du dispositif de pléthysmographie optique à un premier instant et une seconde mesure correspondant à l'intensité lumineuse mesurée par le capteur du dispositif de pléthysmographie optique à un second instant.

3. Procédé selon la revendication 1, dans lequel ledit rapport desdites intensités lumineuses (102 ; 103) est un rapport entre une première mesure correspondant à l'intensité lumineuse mesurée par le capteur du dispositif de pléthysmographie optique à un premier instant et une seconde mesure correspondant à l'intensité lumineuse mesurée par le capteur du dispositif de pléthysmographie optique à un second instant.

4. Procédé selon la revendication 3, dans lequel le premier instant correspond à la diastole dans un premier cycle cardiaque et le second instant correspond à la systole dans un second cycle cardiaque différent du premier cycle cardiaque.

5. Procédé selon la revendication 3, dans lequel le premier instant correspond à la systole dans un premier cycle cardiaque et le second instant correspond à la diastole dans un second cycle cardiaque différent du premier cycle cardiaque.

6. Procédé selon la revendication 3, dans lequel le premier instant correspond à la diastole dans un cycle cardiaque et le second instant correspond à la systole dans un cycle cardiaque.

7. Procédé selon la revendication 3, dans lequel le premier instant correspond à la systole dans un cycle cardiaque et le second instant correspond à la diastole dans un cycle cardiaque.

8. Procédé selon l'une des revendications 1 à 3, dans lequel au moins un (12) desdits instants (12 ; 13) correspond à la diastole dans un cycle cardiaque dudit individu (1) et/ou dans lequel au moins un (13) desdits instants (12 ; 13) correspond à la systole dans un cycle cardiaque dudit individu (1).

9. Procédé selon l'une des revendications précédentes, dans lequel ledit procédé comprend en outre les étapes de :

   - fonctionnement d'une source de lumière (2) configurée pour émettre de la lumière (40) à une longueur d'onde ;

- fonctionnement d'un capteur (4) ;
- collecte, par pléthysmographie optique et sur ledit capteur (4), de la lumière propagée (41) correspondant à la lumière (40) à ladite longueur d'onde qui est transmise ou réfléchie lors de sa propagation dans ledit volume étudié (11) dudit individu (1) auxdits deux instants ou plus (12 ; 13) ; et
- détermination desdites intensités lumineuses (102 ; 103) de ladite lumière propagée sur ledit capteur (4) auxdits deux instants ou plus (12 ; 13).

10. Support de stockage lisible par ordinateur comprenant des instructions exécutables par ordinateur pour effectuer le procédé de l'une quelconque des revendications 1 à 9 lorsque le programme est exécuté sur un ordinateur.

11. Appareil (10) comprenant au moins un processeur et au moins une mémoire (6) incluant un code de programme informatique, l'au moins une mémoire (6) et le code de programme informatique étant configurés pour, avec l'au moins un processeur, amener l'appareil (10) à effectuer le procédé de l'une quelconque des revendications 1 à 9.

12. Appareil selon la revendication 11, dans lequel, lorsque ledit PAT (100) est en dessous de ladite valeur seuil prédéterminée, un événement lié au sommeil est détecté, et dans lequel, lorsque ledit PAT (100) est au-dessus de ladite valeur seuil prédéterminée, aucun événement lié au sommeil n'est détecté.

13. Appareil selon la revendication 12, dans lequel ledit événement lié au sommeil est l'apnée du sommeil.

14. Système (20) comprenant l'appareil selon l'une quelconque des revendications 11 à 13, et comprenant en outre :

- une source de lumière (2 ; 3) configurée pour émettre de la lumière ; et
- le capteur (4) configuré pour collecter par pléthysmographie optique la lumière propagée correspondant à ladite lumière qui est transmise ou réfléchie lors de sa propagation dans ledit volume étudié (11) dudit individu (1) auxdits deux instants ou plus (12 ; 13) ; et configuré en outre pour déterminer lesdites intensités lumineuses (102 ; 103) de ladite lumière propagée auxdits deux instants ou plus (12 ; 13).

Fig. 1

Fig. 2

EP 3 928 690 B1

Fig. 3

Fig. 4

Obtaining:
– an optical plethysmography signal (101) measured at an investigated tissue (11) of said individual (1);
– light intensities (102;103) acquired by optical plethysmography
at two or more points in time (12;13) along said optical plethysmography signal (101)

501

Determining changes in blood volume (16) in said investigated tissue (11)
between said two or more points in time (12;13) by determining a natural logarithm (17)
or a function approximation thereof (17) of a function of said light intensities (102;103),
thereby assessing PAT (100) of said individual (1).

502

Fig. 5

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20060009700 A1

**Non-patent literature cited in the description**

- **NITZAN et al.** The variability of the photoplethysmographic signal - a potential method for the evaluation of the autonomic nervous system. *Physiol. Meas.,* 1998, vol. 19, 93-102
- **HAMUNEN et al.** Effect of pain on autonomic nervous system indices derived from photoplethysmography in healthy volunteers. *British Journal of Anesthesia,* 01 May 2012, vol. 108 (5), 838-844
- **THOMAS PENZEL et al.** Peripheral arterial tonometry, oximetry and actigraphy for ambulatory recording of sleep apnea. *Physiol. Meas.,* 2004, vol. 25, 1025-1036
- **CHATTERJEE et al.** Monte Carlo Analysis of Optical Interactions in Reflectance and Transmittance Finger Photoplethysmography. *Sensors (Basel),* 15 February 2019, vol. 19 (4), 789